# EUROPEAN PATENT SPECIFICATION

(11) **EP 3 006 074 B1**
(45) Date of publication and mention of the grant of the patent: **21.02.2018**
(21) Application number: 13886262.8
(22) Date of filing: 06.06.2013
(51) Int. Cl.: A61M 25/06

(54) **CATHETER ASSEMBLY**
KATHETERANORDNUNG
ENSEMBLE CATHÉTER

(43) Date of publication of application: 13.04.2016
(73) Proprietor: Terumo Kabushiki Kaisha, Tokyo 151-0072 (JP); Mitsubishi Pencil Company, Limited, Tokyo 140-8537 (JP)
(72) Inventor: ISHIDA, Masahiro, Nakakoma-gun Yamanashi 409-3853 (JP)
(74) Representative: Casalonga
(86) International application number: PCT/JP2013/065675
(87) International publication number: WO 2014/196050

(56) References cited:
- EP-A1- 1 475 124
- WO-A1-2013/172104
- JP-A- H1 015 074
- JP-A- H1 157 002
- JP-A- 2001 502 191
- JP-A- 2007 526 059
- US-A1- 2007 179 447
- US-A1- 2015 080 801

## Description

### Technical Field

The present invention relates to a catheter assembly that punctures and remains indwelling in a blood vessel, for example, when performing an infusion on a patient.

### Background Art

Conventionally, when an infusion is carried out on a patient, a catheter assembly, which forms a part for introduction of an infusion into the body, has been used. The catheter assembly, for example, includes a puncturing unit in which an inner needle and an outer needle (catheter) are arranged in an overlapping manner. After a user has punctured and inserted the puncturing unit into the body, the inner needle is withdrawn from the catheter, and an infusion line is constructed that administers an infusion agent via the catheter into the body.

On the other hand, at a time that the inner needle is withdrawn from the catheter, by being accommodated in a needle cover of the catheter assembly, safety is assured when the inner needle is withdrawn. For example, in Japanese Laid-Open Patent Publication No. 2007-526059 (PCT), a catheter assembly is disclosed which is equipped with a cage (needle cover) that accommodates a distal end portion of an inner needle when the inner needle is withdrawn. The catheter assembly includes a retaining member that is attached rotatably to the needle cover. By rotating the retaining member after the inner needle has been withdrawn, the retaining member is arranged so as to face the inner needle, so that advancement (reversal in movement) of the inner needle is blocked.

Further, in Japanese Patent No. 2921561, a catheter assembly is disclosed which is equipped with another structure (a plate spring and a locking pin) for preventing advancement of the inner needle. The catheter assembly blocks advancement of the inner needle by displacing the locking pin and pressing the distal end portion of the inner needle by an elastic force of the plate spring accompanying withdrawal of the inner needle.

Incidentally, in the catheter assemblies disclosed in the aforementioned Japanese Laid-Open Patent Publication No. 2007-526059 (PCT) and Japanese Patent No. 2921561, configurations are provided in which a stopper portion (the retaining member, the plate spring and locking pin), which make up structures for blocking advancement of the inner needle, are members that are separate from the needle cover. More specifically, by fabricating the stopper portions separately in the manufacturing process for such catheter assemblies, the manufacturing cost tends to increase. Furthermore, the stopper portions are formed to be small to match with the size of the needle cover. Therefore, during the assembly of such catheter assemblies, time is expended in assembling the stopper portions, with the disadvantage that production efficiency is lowered significantly. See as well US2007179447.

### Summary of Invention

The present invention has been devised in consideration of the aforementioned circumstances, and has the object of providing a catheter assembly in which, by integrally forming a stopper portion that blocks advancement of the inner needle slidably on a needle cover, manufacturing costs can be reduced and production efficiency can be enhanced.

For achieving the aforementioned object, a catheter assembly of the present invention, as claimed in claim 1, is equipped with an inner needle, a catheter through which the inner needle is inserted, and a needle cover that accommodates the inner needle when the inner needle is withdrawn from the catheter. The needle cover includes an inner tube that is capable of being attached to and detached from a catheter hub configured to retain the catheter, and an outer tube configured to cover at least a portion of the inner tube, and to move relative to the inner tube when the inner needle is withdrawn. The inner tube includes a stopper portion configured to prevent relative movement between the inner tube and the outer tube by being disposed in a standby position proximate a radially outer side of the inner tube, in a state in which the inner needle has been inserted through the catheter, and the stopper portion is configured to slide to an inner needle blocking position accompanying relative movement of the outer tube with respect to the inner tube when the inner needle is withdrawn.

According to the above, in the catheter assembly, by sliding and arranging the stopper portion that prevents relative movement between the inner tube and the outer tube at the inner needle blocking position upon withdrawal of the inner needle, in a condition after withdrawal of the inner needle, advancement of the inner needle can suitably be prevented by the stopper portion. With the structure by which the stopper portion is slidable, the stopper portion can be formed with a small size, the amount of movement thereof can be made small, and the catheter assembly can be reduced in size. Further, by the inner tube of the catheter assembly having the stopper portion, there is no need for a member that blocks the inner needle to be prepared as a separate member, and assembly thereof to an inner portion is rendered unnecessary. Therefore, the production cost for the catheter assembly can be reduced significantly, the assembly operation can be simplified, and production efficiency can be enhanced.

In this case, a configuration may be provided in which the stopper portion includes an inclined surface that is inclined with respect to an axial direction of the inner tube, and the inclined surface is configured to slide to the inner needle blocking position by coming into contact with and being pressed by a portion of the outer tube when there is relative movement of the outer tube with respect to the inner tube.

In this manner, by the stopper portion having the inclined surface, during relative movement of the outer tube, the stopper portion slides smoothly along the inclined surface that is in contact with a wall portion of the outer tube. Thus, the operation to withdraw the inner needle can easily be carried out.

Further, the outer tube preferably includes a hollow portion through which the inner tube is inserted, and the inclined surface is configured to be inserted into the hollow portion in a state in which the stopper portion is disposed in the standby position.

In this manner, by insertion of the inclined surface into the interior of the hollow portion of the outer tube, at the start of relative movement of the outer tube, the inclined surface can be brought into contact with the wall portion that makes up the hollow portion. Consequently, sliding of the stopper portion can be guided more easily.

Furthermore, a configuration may be provided in which the stopper portion includes a reverse side inclined surface disposed at an opposite position to the inclined surface, so that in a state in which the inner needle extends through the catheter, sliding of the stopper portion radially-inwardly from the standby position is restricted by the inner needle, and the outer tube comprises a wall portion configured to be displaceable radially-outwardly with respect to the outer tube on a more distal end side than the stopper portion, and the wall portion includes an outer tube side inclined surface that is substantially parallel with the reverse side inclined surface.

Consequently, in the catheter assembly, when the outer tube and the inner tube are attached, the reverse side inclined surface of the stopper portion, for which sliding thereof is restricted by the inner needle, comes into contact with the outer tube side inclined surface of the outer tube. In addition, since by movement of the inner tube in the direction of insertion, the reverse side inclined surface can cause displacement of the wall portion of the outer tube, mutual assembly of the inner tube and the outer tube can be performed easily.

The inner tube and the stopper portion preferably are connected by a connecting part including a hinge mechanism that is configured to allow the stopper portion to freely slide.

In this manner, by connecting the inner tube and the stopper portion by way of the connecting part, integral formation and assembly thereof can easily be performed. Further, since the connecting part includes the hinge mechanism, the stopper portion is capable of sliding suitably with respect to the inner tube.

Further, a groove that extends along a direction in which the stopper portion slides may be formed on one of the inner tube and the stopper portion, and a protrusion, which is inserted into the groove, may be formed on another of the inner tube and the stopper portion.

In this manner, in the catheter assembly, by the groove or the protrusion being formed on the inner tube and the stopper portion, an inclination or tilting in the posture of the stopper portion can be prevented.

Furthermore, a lock mechanism configured to maintain a displaced condition when the stopper portion has slid to the inner needle blocking position may be disposed in the inner tube and the stopper portion.

As a result of the lock mechanism, after sliding, the stopper portion can reliably be made to continue waiting in the inner needle blocking position, and flying out, etc., of the inner needle that is accommodated in the needle cover can reliably be blocked.

Still further, the inner tube may comprise a first member that includes the stopper portion, a space that surrounds the stopper portion in a non-contacting manner, and a second member that is attached to the first member. In addition, the second member may include projecting walls that are accommodated in the space so that gaps on both sides of the stopper portion are narrow in width.

In the foregoing manner, in the catheter assembly, due to the inner tube comprising the first member and the second member, the shape thereof including the stopper portion can easily be formed by injection molding. Further, upon assembling the inner tube, the projecting walls can be made to surround the periphery of the stopper portion, and by the gaps being reduced, flying out of the inner needle can more reliably be prevented.

Further still, the stopper portion may include a projecting part that surrounds a portion of an accommodating space for the inner needle in the inner needle blocking position, and the projecting walls may be arranged so that gaps on both sides of the projecting part also are narrow in width.

In the catheter assembly, by providing the projecting part and the projecting walls, since the gaps of the accommodating space for the inner needle are small and can be blocked, flying out of the inner needle can more reliably be prevented.

### Brief Description of Drawings

FIG. 1 is a perspective view showing an overall structure of a catheter assembly according to an embodiment of the present invention;
FIG. 2 is a plan view of the catheter assembly shown in FIG. 1;
FIG. 3A is a first cross-sectional view for describing operations of the catheter assembly of FIG. 1;
FIG. 3B is a second cross-sectional view for describing operations of the catheter assembly of FIG. 1;
FIG. 3C is a third cross-sectional view for describing operations of the catheter assembly of FIG. 1;
FIG. 4 is a partial perspective view showing a condition in which a catheter and a catheter hub are separated, in the catheter assembly of FIG. 1;
FIG. 5A is a partially enlarged cross-sectional view for describing the structure of a stopper portion;
FIG. 5B is a partially enlarged perspective view corresponding to FIG. 5A;
FIG. 6A is a plan view of the stopper portion;
FIG. 6B is a side view of the stopper portion;
FIG. 6C is a rear view of the stopper portion;
FIG. 7A is a cross-sectional view taken along line VIIA-VIIA of FIG. 5B;
FIG. 7B is a cross-sectional view continuing from FIG. 7A for describing a hook part;
FIG. 8 is a perspective view for describing assembly of a first member and a second member of an inner tube;
FIG. 9 is a partial side cross-sectional view for describing assembly of the inner tube and an outer tube;
FIG. 10A is a partially enlarged cross-sectional view continuing from FIG. 5A for describing operations of the stopper portion;
FIG. 10B is a partially enlarged perspective view corresponding to FIG. 10A;
FIG. 11A is a partially enlarged cross-sectional view continuing from FIG. 10A for describing operations of the stopper portion;
FIG. 11B is a partially enlarged perspective view corresponding to FIG. 11A;
FIG. 12A is a partially enlarged cross-sectional view for describing operations of the stopper portion in an inner needle accommodating state; and
FIG. 12B is a partially enlarged plan view for describing operations of the stopper portion in the inner needle accommodating state.

### Description of Embodiments

Hereinafter, a preferred embodiment of a catheter assembly according to the present invention will be described in detail below with reference to the accompanying drawings.

The catheter assembly according to the present invention is used as a connecting portion that is connected to the patient in an infusion line when an infusion on the patient is carried out. Therefore, when the infusion line is constructed, the catheter assembly is constituted so that a portion thereof that has punctured the patient remains indwelling in the patient, and thereafter (during the infusion), an infusion agent is introduced into the body through the indwelling portion.

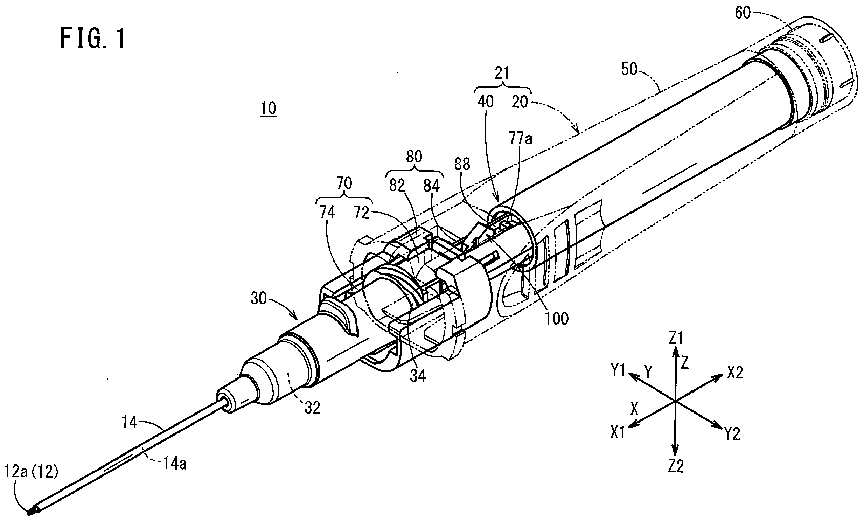

As shown in FIG. 1, a catheter assembly 10 includes a tubular shaped inner needle 12 having a sharp distal end tip 12a that serves as a puncturing unit that pierces the body of the patient, and a catheter 14 constituted as an outer needle through which the inner needle 12 is inserted. The catheter assembly 10 is further equipped with an inner needle hub 20 that retains the inner needle 12 and serves as an operating unit when puncturing is performed, a catheter hub 30 that retains the catheter 14, and a needle cover 40 that covers the tip 12a of the inner needle 12 when the inner needle 12 is withdrawn or retracted.

In the following description, directions of the catheter assembly 10 will also be indicated based on the direction indications shown in FIG. 1. More specifically, an axial direction of the catheter assembly 10 will be referred to as an X direction (the distal end direction at the tip 12a is referred to as an X1 direction, the proximal end direction at the proximal end of the inner needle hub 20 is referred to as an X2 direction). Further, the widthwise direction of the catheter assembly 10 will be referred to as a Y direction (an oblique leftward direction in FIG. 1 is referred to as a Y1 direction, an oblique rightward direction is referred to as a Y2 direction). Further, the vertical direction of the catheter assembly 10 will be referred to as a Z direction (an upward direction in FIG. 1 is referred to as a Z1 direction, a downward direction is referred to as a Z2 direction). The aforementioned directions are for facilitating descriptions, and it goes without saying that the catheter assembly 10 can be used in any arbitrary orientation.

Below, for simplifying understanding of the present invention, a method of using the catheter assembly 10 will be described below in outline. A user (a doctor or a nurse, etc.) performs an operation to grip the inner needle hub 20 of the catheter assembly 10, whereby the puncturing unit thereof carries out a puncturing operation. In an initial condition prior to use of the puncturing unit (before puncturing the patient), the inner needle 12 is inserted through the catheter 14 in the form of a double tube structure, and the inner needle 12 projects a predetermined length from the distal end of the catheter 14. Hereinbelow, the initial condition of the catheter assembly 10 will also be referred to as a "puncture enabled state". Further, in the initial condition of the catheter assembly 10, the inner needle hub 20 and the catheter hub 30 are connected through the needle cover 40.

In the puncture enabled state of the catheter assembly 10, by a puncturing act performed by the user, the inner needle 12 and the catheter 14 are inserted together into the blood vessel of the patient. After puncturing of the patient, the user fixes the puncture position of the catheter 14 and the catheter hub 30, and by moving the inner needle hub 20 in a retracting direction so as to be pulled out from the patient, withdrawal of the inner needle 12 is carried out. Consequently, the inner needle 12 and the needle cover 40, which are retained by the inner needle hub 20, also are moved in the retracting direction, whereupon the catheter hub 30 and the needle cover 40 are detached from each other.

As a result, in the catheter assembly 10, the inner needle 12, the inner needle hub 20, and the needle cover 40 are separated from the side of the patient, and a state is brought about in which only the catheter 14 and the catheter hub 30 are left indwelling on the side of the patient. After the inner needle 12 has been withdrawn, a non-illustrated connector of an infusion tube is connected to the proximal end side of the catheter hub 30, whereby an infusion line is constructed, and supply of an infusion agent (medicinal solution) is performed on the patient.

On the other hand, when the inner needle 12 is subjected to a withdrawing operation, in the inner needle hub 20 and the needle cover 40, an operation to accommodate the inner needle 12 is performed. In this case, the inner needle cover 40 is expanded in length with respect to the inner needle hub 20, and by accommodating the inner needle 12 in the interior of the inner needle hub 20 and the needle cover 40, extruding of the inner needle 12 to the outside is prevented. Below, the condition of the inner needle hub 20 and the needle cover 40 following withdrawal of the inner needle 12 will also be referred to as a "withdrawn state".

Respective structures of the catheter assembly 10 will be described in detail below with reference to FIG. 1, FIG. 2, FIGS. 3A to 3C, and FIG. 4.

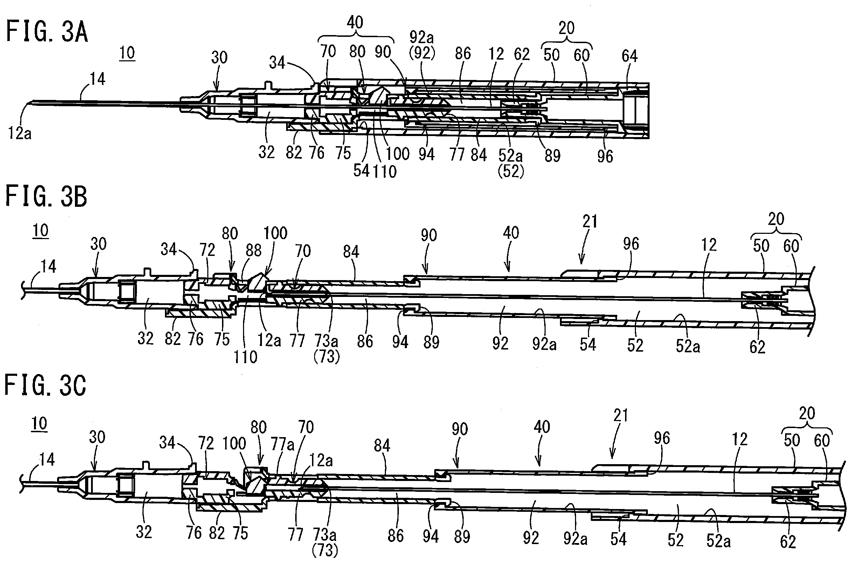

The inner needle 12 of the catheter assembly 10 is a rigid tubular member that is capable of puncturing the patient's skin. In the puncture enabled state (see FIG. 3A), the inner needle 12 is formed with a length that enables the tip 12a of the inner needle 12 to project out from the distal end opening of the catheter 14, and enables an intermediate region in the longitudinal direction of the inner needle 12 to be inserted through the interior of the catheter hub 30, and enables the proximal end side of the inner needle 12 to be retained in the interior of the inner needle hub 20. As a constituent material of the inner needle 12, a metal material, for example, such as stainless steel, aluminum or aluminum alloy, titanium or titanium alloy, may be used.

The catheter 14, which constitutes the puncturing unit together with the inner needle 12, is a flexible narrow diameter tubular member, and is formed with a length to allow introduction and indwelling thereof reliably in the body of the patient. A lumen 14a that extends in the axial direction of the catheter 14 is formed to penetrate through the interior of the catheter 14. The lumen 14a has an inner diameter that enables the inner needle 12 to be inserted through the lumen 14a.

As the material composing the catheter 14, a resin, particularly, a soft resin material is preferred. In this case, for example, a fluororesin such as polytetrafluoroethylene (PTFE), ethylene-tetrafluoroethylene copolymer (ETFE), perfluoroalkoxy fluororesin (PFA), an olefin resin such as polyethylene and polypropylene or a mixture thereof, polyurethane, polyester, polyamide, polyether nylon resin, and a mixture of the olefin resin and ethylene-vinyl acetate copolymer may be used. The catheter 14 may be constituted from a transparent resin material, so that all or a portion of the interior thereof is visible.

The catheter hub 30 is connected in a fixed manner to the proximal end of the catheter 14. The catheter hub 30 is formed in a tapered tubular shape, and has an interior space 32 corresponding to the outer form of the tubular shape. In the interior space 32, in the puncture enabled condition (see FIG. 3A), the inner needle 12 is arranged along the axial direction of the catheter hub 30. In the distal end side of the interior space 32, a non-illustrated connecting mechanism (for example, a crimping pin) is provided that connects in a liquid-tight manner the distal end portion of the catheter hub 30 and the proximal end portion of the catheter 14. A flange 34, which projects outwardly and extends in a circumferential direction, is provided on an outer circumferential surface on the proximal end of the catheter hub 30.

When the catheter assembly 10 is used, the catheter hub 30 remains on the patient's body surface in a state in which the catheter 14 has pierced into the blood vessel, and is pasted and held in place on the skin by tape or the like. The catheter hub 30 preferably is constituted from a material that is more rigid than the catheter 14. The constituent material of the catheter hub 30 is not limited to any particular material, however, a thermoplastic resin material, for example, polypropylene, polycarbonate, polyamide, polysulfone, polyarylate, methacrylate-butylene-styrene copolymer, etc., preferably can be used.

Further, when the catheter hub 30 is left indwelling on the side of the patient, a connector of the aforementioned infusion tube is connected to the catheter hub 30. Therefore, preferably, a non-illustrated connection mechanism (e.g., a valve member, a seal member, and a plug, etc.) is accommodated in the interior space 32, by which a passage of the infusion line is constructed accompanying insertion of the connector therein.

On the other hand, in the puncture enabled state, the inner needle hub 20 of the catheter assembly 10 partially covers the proximal end side of the catheter hub 30. Further, in the withdrawn state, the inner needle hub 20 functions as part of an accommodating member 21 that, together with the needle cover 40, accommodates the inner needle 12. Stated otherwise, the accommodating member 21 is constituted from the inner needle hub 20 and the needle cover 40. The inner needle hub 20 is equipped with a hub main body 50 that is gripped by the user and constitutes a proximal end side of the catheter assembly 10, and a hollow inner needle fixing member 60 that is fitted into the proximal end side of the hub main body 50.

The hub main body 50 is a long and narrow tubular body (outer sleeve) and is formed with an appropriate size (thickness, length) to enable the hub main body 50 to be gripped and operated easily by the user when using the catheter assembly 10. In the interior of the hub main body 50, an inner needle hub side hollow portion 52 is provided that accommodates the needle cover 40. Proximate the distal end of an inner circumferential surface 52a that constitutes the inner needle hub side hollow portion 52, a hub main body side protrusion 54 is disposed that restricts detachment and separation of the needle cover 40 (an adjoining tube 90) when the withdrawal operation of the inner needle 12 is performed.

The inner needle fixing member 60 is accommodated and fixed on a proximal end side of the inner needle hub side hollow section 52. The inner needle fixing member 60 exhibits a tubular shape that narrows in diameter stepwise toward the distal end side in the interior of the inner needle hub side hollow section 52. The distal end portion, which is smallest in diameter, is constituted as a retaining part 62 that serves to retain (tightly fix) the proximal end side of the inner needle 12, whereas the proximal end portion, which is largest in diameter, is constituted as a fitting part 64 that is fitted on the inner circumferential surface of the hub main body 50.

In the puncture enabled state (see FIG. 3A), a large part of the proximal end side of the needle cover 40 of the catheter assembly 10 is accommodated in the inner needle hub 20, and the catheter hub 30 is retained at the distal end side. The needle cover 40 is constituted from the three tubular members of an inner tube 70, an outer tube 80, and the adjoining tube 90. The inner tube 70, the outer tube 80, and the adjoining tube 90 are designed such that the shapes of the respective tubular parts thereof gradually become larger in diameter, and are assembled together such that the axes of the tubular portions are mutually coaxial.

More specifically, the proximal end side of the inner tube 70 is inserted into the interior of the outer tube 80, and is movable in the axial direction relative to the outer tube 80. Further, the proximal end side of the outer tube 80 is inserted into the interior of the adjoining tube 90, and is movable in the axial direction relative to the adjoining tube 90. Furthermore, the entirety of the adjoining tube 90 is inserted into the interior of the hub main body 50, and is movable in the axial direction relative to the hub main body 50. By the inner tube 70 being relatively movable with respect to the outer tube 80, the outer tube 80 being relatively movable with respect to the adjoining tube 90, and the adjoining tube 90 being relatively movable with respect to the hub main body 50, respectively, the needle cover 40 is expanded to a length that enables the inner needle 12 to be accommodated therein together with the hub main body 50.

The inner tube 70 of the needle cover 40 functions as a tip protecting member that covers the tip 12a of the inner needle 12 when the inner needle 12 is accommodated therein. In the puncture enabled state (see FIG. 3A), the distal end of the inner tube 70 is at a more proximal position than the distal end of the outer tube 80, and is displaced to project out from the distal end of the outer tube 80 accompanying the withdrawal operation of the inner needle 12.

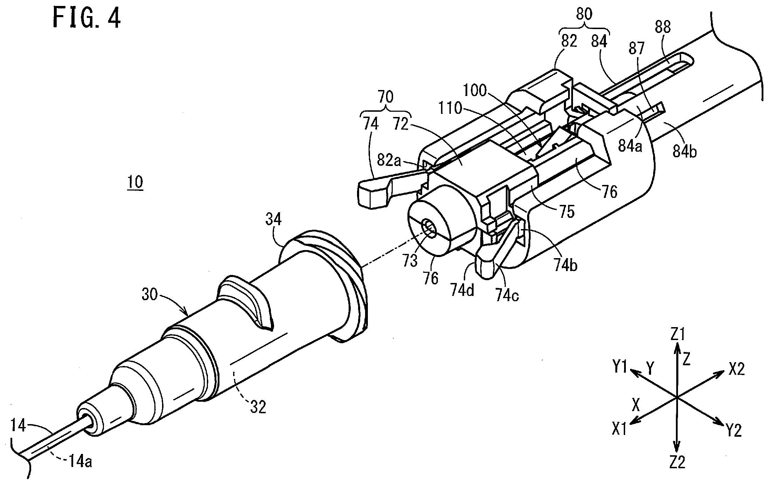

As shown in FIGS. 3A and 4, the inner tube 70 is equipped with an inner tube main body 72 having a predetermined length in the axial direction, and arms 74 that are formed integrally with the inner tube main body 72. Further, an insertion hole 73 (accommodating space) through which the inner needle 12 can pass is formed in the interior of the inner tube main body 72 along the axial direction of the inner tube main body 72.

The inner tube main body 72 includes an intermediate tubular section 75 to which the arms 74 are connected, a distal end tubular section 76 that projects out from the intermediate tubular section 75 on the distal end side thereof, and a proximal end tubular section 77 that projects out from the intermediate tubular section 75 on the proximal end side thereof. The intermediate tubular section 75 is formed with a size that is larger than the distal end tubular section 76 and the proximal end tubular section 77, and the arms 74 are connected integrally on opposite side portions in the widthwise direction (Y direction) thereof.

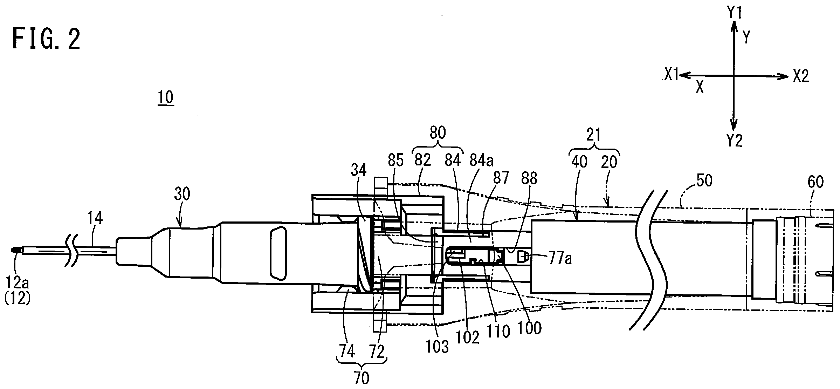

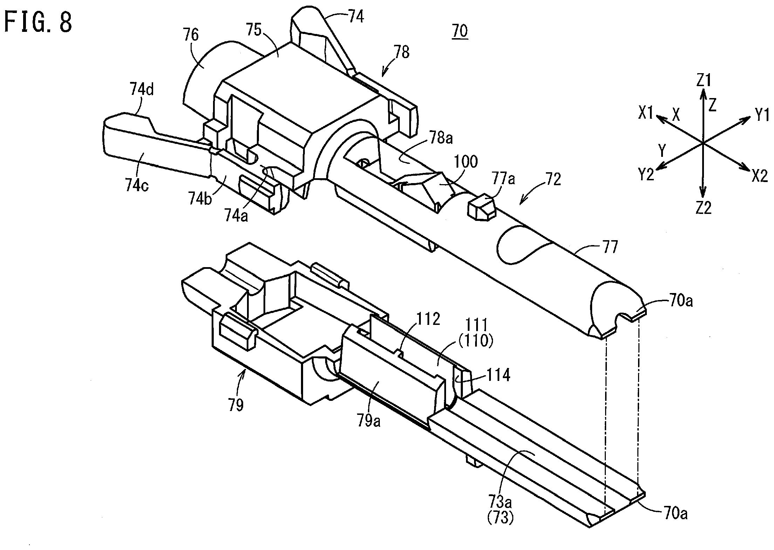

Each of the pair of arms 74 includes a supporting portion 74a (see FIG. 8) contiguous with the intermediate tubular section 75, an arm base portion 74b contiguous with the supporting portion 74a and which extends in parallel with the distal end direction, and an arm projecting portion 74c that is contiguous with the arm base portion 74b and projects in an outside widthwise direction and in the distal end direction. In a natural state in which external forces do not act thereon, as shown in FIG. 4, the arm projecting portions 74c of the arms 74 are formed to widen outwardly in the widthwise direction more than the outer tube 80. In addition, in the puncture enabled state when the inner tube 70 and outer tube 80 are overlapped, the arms 74 are pressed elastically so as to approach one another inwardly by the side wall of the outer tube 80 (see FIG. 2).

On distal end portions of the pair of arm projecting portions 74c, protrusions 74d are provided that protrude inwardly in the widthwise direction. In the puncture enabled state, by the arm projecting portions 74c being brought closer inwardly, the two protrusions 74d (in pairs) become caught on the flange 34 of the catheter hub 30. Consequently, in the inner tube 70, the catheter hub 30 is sandwiched between the protrusions 74d and the distal end tubular section 76 that is inserted on the proximal end side of the interior space 32 of the catheter hub 30, and the inner tube 70 is connected to and forcibly retains the catheter hub 30. When the withdrawing operation of the inner needle 12 is performed, the inner tube 70 and the outer tube 80 move relatively to one another, whereupon by displacement of the inner tube 70 toward the distal end side of the outer tube 80, the two arm projecting portions 74c are expanded outwardly and elastically, and catching of the flange 34 by the protrusions 74d is released. As a result, detachment of the catheter hub 30 from the inner tube 70 (needle cover 40) is permitted.

Referring back to FIGS. 3A to 3C, the distal end tubular section 76 of the inner tube 70 is formed in a cylindrical shape having an outer diameter that matches substantially with the inner diameter of the interior space 32 of the catheter hub 30. On the other hand, the proximal end tubular section 77 of the inner tube 70 is formed in a cylindrical shape that is longer in the axial direction than the distal end tubular section 76, and is smaller in diameter than the inner diameter of an outer tube side hollow portion 86 of the outer tube 80. In the puncture enabled state, a stopper portion 100 that prevents detachment of the inner tube 70 and the outer tube 80, and a displacement permitting space 110 that enables displacement of the stopper portion 100 are provided on a distal end side of the proximal end tubular section 77 (at a connecting location between the intermediate tubular section 75 and the proximal end tubular section 77). The configuration thereof forms an essential characteristic part of the present invention, which will be described in detail later.

Further, an inner tube side projection 77a is formed on the outer circumferential surface more toward the proximal end side of the proximal end tubular section 77 than the stopper portion 100. The inner tube side projection 77a is inserted into an elongate hole 88 of the outer tube 80 in the puncture enabled state, and at a time of relative movement between the inner tube 70 and the outer tube 80, restricts relative displacement of the inner tube 70 and the outer tube 80 beyond a predetermined amount. Owing thereto, complete pulling out of the inner tube 70 from the outer tube 80 is prevented.

The outer tube 80 of the needle cover 40 is provided outside of the inner tube 70 and functions as a tip protecting member that covers the distal end proximity of the inner needle 12 when the inner needle 12 is accommodated therein. The outer tube 80 includes an arm accommodating section 82 in which the arms 74 can be accommodated at distal end sides thereof, and a tubular portion 84 that extends a predetermined length in the proximal end direction from the proximal end of the arm accommodating section 82. Further, the outer tube side hollow portion 86 is formed to penetrate in the axial direction through the interior of the tubular portion 84.

The arm accommodating section 82 is formed in a comparatively large box-like shape that opens on the upper part and the distal end portion thereof. In the puncture enabled state of the catheter assembly 10, the proximal end of the catheter hub 30 and the distal end side of the inner tube 70 (the pair of arms 74 and the intermediate tubular section 75) are arranged in the interior of the arm accommodating section 82. Recessed grooves 82a, which open on distal end sides thereof and are formed parallel to the axial direction of the outer tube 80, are provided on both side surfaces in the Y direction of the arm accommodating section 82. The arms 74 are inserted slidably into the recessed grooves 82a, such that when the inner tube 70 and the outer tube 80 are moved relatively, the arms 74 are guided by the recessed grooves 82a.

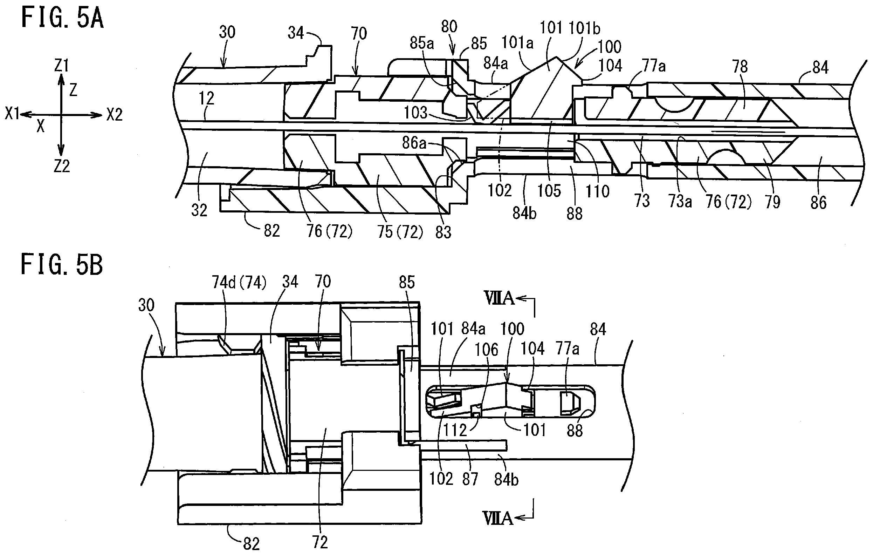

As shown in FIGS. 5A and 5B, a proximal end wall 83 of the arm accommodating section 82 is formed substantially in a U-shape as viewed from the front, so as to surround an opening 86a of the outer tube side hollow portion 86. The proximal end wall 83 is contiguous with the tubular portion 84 on the proximal end side. More specifically, the opening 86a of the outer tube side hollow portion 86 is made up from the proximal end wall 83 of the arm accommodating section 82 on the lower side, and a distal end wall 85 (wall portion) on the upper side that is joined to the tubular portion 84. A pair of slits 87 that divide the distal end wall 85 from the proximal end wall 83 are formed on the side circumferential surfaces of the tubular portion 84.

The tubular portion 84 is formed with an axial length that is longer than the proximal end tubular portion 77 of the inner tube 70. The aforementioned slits 87 cut into the distal end side of the tubular portion 84 for a predetermined length, and as a result, the distal end side of the tubular portion 84 is divided into an upper wall portion 84a and a lower wall portion 84b. In addition, the aforementioned distal end wall 85 is disposed on the distal end of the upper wall portion 84a.

The distal end wall 85 overlaps with an axial direction forming position of the proximal end wall 83 of the arm accommodating section 82, and on an opening edge that makes up the opening 86a of the outer tube side hollow portion 86, a tapered surface 85a (outer tube side inclined surface) is included that inclines in the proximal end direction from the distal end face. Further, at a position that vertically faces the upper wall portion 84a and the lower wall portion 84b, the elongate hole 88 with a predetermined length in the axial direction is formed. The elongate hole 88 communicates with the outer tube side hollow portion 86.

As shown in FIGS. 3A to 3C, an outer tube side projection 89 that projects radially-outwardly is formed along the circumferential direction on the outer circumferential surface on the proximal end side of the tubular portion 84. In a state in which the tubular portion 84 is inserted into the adjoining tube 90, the outer tube side projection 89 is capable of becoming caught on an adjoining tube inner side projection 94 provided on the distal end portion of the adjoining tube 90. Owing thereto, at a time of relative movement between the outer tube 80 and the adjoining tube 90, relative displacement of the outer tube 80 and the adjoining tube 90 beyond a predetermined amount is restricted, and complete pulling out of the outer tube 80 from the adjoining tube 90 is prevented.

The adjoining tube 90 of the needle cover 40 is provided outside of the outer tube 80 and functions as a tip protecting member that covers a central body portion of the inner needle 12 when the inner needle 12 is accommodated therein. An adjoining tube side hollow portion 92 is formed to penetrate in the axial direction through the interior of the adjoining tube 90. The aforementioned adjoining tube inner side projection 94 is formed on an inner circumferential surface that makes up the adjoining tube side hollow portion 92 on the distal end of the adjoining tube 90.

Further, an adjoining tube outer side projection 96 that projects radially-outwardly is formed along the circumferential direction on the outer circumferential surface on the proximal end side of the adjoining tube 90. In a state in which the adjoining tube 90 is inserted into the inner needle hub side hollow portion 52 of the hub main body 50, the adjoining tube outer side projection 96 is capable of becoming caught on an adjoining hub main body side projection 54 of the hub main body 50. Owing thereto, at a time of relative movement between the adjoining tube 90 and the inner needle hub 20, relative displacement of the adjoining tube 90 and the hub main body 50 beyond a predetermined amount is restricted, and complete pulling out of the adjoining tube 90 from the hub main body 50 is prevented.

The needle cover 40, as a result of being constructed in the foregoing manner, allows the inner needle 12 to protrude out from the distal end of the catheter 14 in the puncture enabled state, and accommodates the inner needle 12 in the interior thereof in the withdrawn state of the inner needle 12. In addition, with the catheter assembly 10 according to the present embodiment, in the puncture enabled state, the stopper portion 100 is disposed in the puncture standby position near the radially outer side of the inner tube 70, whereby disengagement between the inner tube 70 and the outer tube 80 is prevented. Further, by the stopper portion 100 being disposed in the inner needle blocking position of the inner tube 70 in a state after withdrawal of the inner needle 12, advancement of the inner needle 12 (flying out of the tip 12a) that is accommodated in the interior of the needle cover 40 is blocked. Below, the structure of the stopper portion 100, together with structures of parts in the vicinity thereof, will be described in greater detail.

As shown in FIG. 5A, the stopper portion 100 is arranged in the displacement permitting space 110 of the inner tube 70. The displacement permitting space 110 is formed to penetrate in a direction (Z direction) perpendicular to the axial direction of the inner tube 70 on the distal end side of the proximal end tubular section 77, and communicates with the insertion hole 73 of the inner tube 70 that extends in the axial direction. In the displacement permitting space 110, the stopper portion 100 is arranged slidably in the vertical direction of the inner tube 70.

In the puncture enabled state (a state in which the inner needle 12 passes through the insertion hole 73), the stopper portion 100 is pressed out by the side surface of the inner needle 12, and is disposed in the puncture standby position (on an upper part of the displacement permitting space 110) of the inner tube 70. At this time, as shown in FIG. 3A, the stopper portion 100 projects out of the elongate hole 88 of the outer tube 80, protrudes outside of the inner needle hub side hollow portion 52 of the inner needle hub 20, and resides between the inner needle 12 and the inner circumferential surface that makes up the inner needle hub side hollow portion 52.

Therefore, for example, even if the inner tube 70 is moved in the distal end direction relative to the outer tube 80, the stopper portion 100 abuts against the hole edge of the elongate hole 88, and advancement or forward movement of the inner tube 70 from the outer tube 80 can be prevented. In other words, when the withdrawal operation of the inner needle 12 is performed, the stopper portion 100 is maintained in the puncture standby position, until the inner needle 12 is completely pulled out in the proximal end direction from the displacement permitting space 110. The connection between the inner tube 70 and the outer tube 80 continues until the outer tube 80, the adjoining tube 90, and the hub main body 50 are extended sufficiently in the axial direction. After the inner needle 12 has been moved and retracted to the proximal end side beyond the displacement permitting space 110, the stopper portion 100 becomes capable of sliding in a downward direction of the displacement permitting space 110, whereby the stopper portion 100 undergoes sliding accompanying relative movement between the inner tube 70 and the outer tube 80. In addition, by the distal end of the outer tube 80 being retracted and the arms 74 of the inner tube 70 being allowed to open, the catheter hub 30 is disengaged from the inner tube 70.

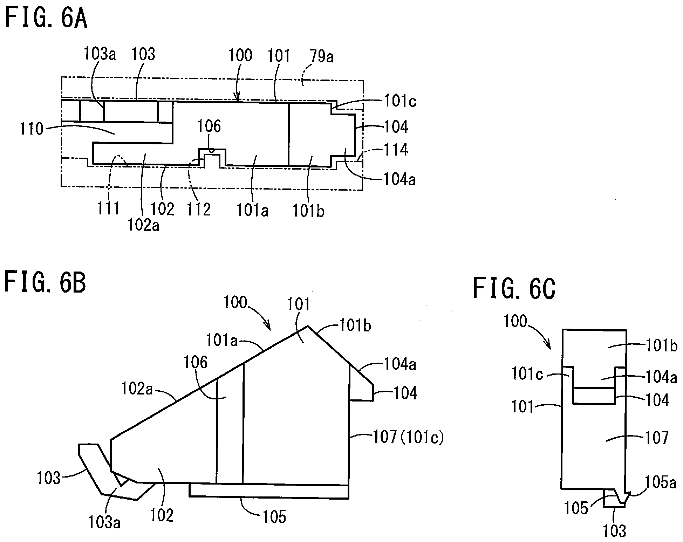

The shape of the stopper portion 100 will be described in detail below with reference to FIGS. 6A to 6C. The stopper portion 100 includes a block-shaped base portion 101 that exhibits a predetermined shape, a projecting piece 102 and a connecting part 103 that project out from a distal end of the base portion 101, a proximal end projecting part 104 that projects out from the proximal end of the base portion 101, and a hook part 105 that projects out from a lower end of the base portion 101.

An upper part of the base portion 101 is formed in a chevron shape as viewed from the side. The base portion 101 includes a distal end inclined surface 101a, which is inclined in a downward distal end direction from an apex of the chevron shape, and a proximal end inclined surface 101b (reverse side inclined surface), which is inclined in a downward proximal end direction from the apex. The width of the base portion 101 is formed to be more narrow than the displacement permitting space 110, yet to be larger than an inner diameter of the insertion hole 73 of the inner tube 70. Further, the height from the apex to the lower end of the base portion 101 is set to be higher than the vertical height of the displacement permitting space 110.

A guide groove 106 (groove member) is formed along the vertical direction on a side surface (a side surface contiguous with the projecting piece 102) in the Y2 direction of the base portion 101. A sideways protrusion 112, which is formed on an inside surface 111 that constitutes the displacement permitting space 110, is inserted into the guide groove 106. As a result thereof, at the time of sliding, since the guide groove 106 is guided by the sideways protrusion 112, the posture of the stopper portion 100 is prevented from inclining to the axial direction of the inner tube 70.

The projecting piece 102 is contiguous to a side surface in the Y2 direction of the base portion 101, and is formed in a substantially triangular shape that projects to a predetermined position on the distal end side of the displacement permitting space 110. The width of the projecting piece 102 is set to a dimension that is narrower in width than the width of the base portion 101. An upper surface 102a of the projecting piece 102 is contiguous to and planar with the inclination of the distal end inclined surface 101a. More specifically, even in the case that the stopper portion 100 is in the puncture standby position shown in FIG. 5A, the distal end of the distal end inclined surface 101a is lowered to a position that enters into the outer tube side hollow portion 86 of the outer tube 80.

On the other hand, the connecting part 103 serves to connect the stopper portion 100 to the inner tube 70, so that during molding of the inner tube 70, the stopper portion 100 can be molded integrally therewith. More specifically, the connecting part 103 is connected to a side in the Y1 direction of the distal end face of the base portion 101, and is continuous with a wall surface of the proximal end tubular section 77 that makes up the displacement permitting space 110 of the inner tube 70.

The connecting part 103 is formed in a V-shape as viewed from the side, and at a connecting site at the base portion 101, and at connecting sites at a valley location and a wall surface, hinge mechanisms 103a are constructed so as to make portions therebetween freely swingable. By the respective hinge mechanisms 103a of the connecting part 103 being linked mutually with each other, in the interior of the displacement permitting space 110, sliding in the vertical direction of the stopper portion 100 can be implemented stably.

The proximal end projecting part 104 of the stopper portion 100 is of a degree that projects out slightly in the proximal end direction from a proximal end surface 101c of the base portion 101, and is disposed in the proximity of an upper portion of the proximal end surface 101c of the base portion 101. An upper surface 104a of the proximal end projecting part 104 is contiguous to and planar with the inclination of the proximal end inclined surface 101b of the base portion 101.

Further, a side of the proximal end surface 101c that is lower than the proximal end projecting part 104, is formed in a flat shape, and the surface shape (width and height) thereof is set to be larger than the inner diameter of the insertion hole 73. A portion of the side of the proximal end surface 101c that is lower than the proximal end projecting part 104 faces toward the insertion hole 73 of the inner tube 70, in a condition in which the stopper portion 100 is disposed at the inner needle blocking position. Below, such a portion may also be referred to as a "facing surface 107".

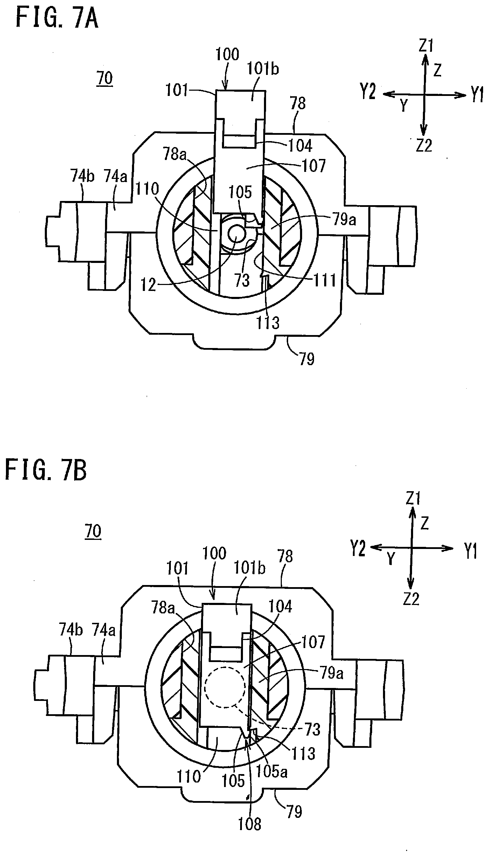

The hook part 105 of the stopper portion 100 is disposed at a position contiguous with a lower end surface and a side surface in the Y1 direction of the base portion 101, and is formed to extend over the base portion 101 in the axial direction. The hook part 105 includes a claw member 105a that projects in the Y1 direction, such that when the stopper portion 100 is slid downwardly inside the displacement permitting space 110, the claw member 105a becomes hooked in a notch 113. More specifically, the hook part 105 and the notch 113 make up a lock mechanism 108 (see FIG. 7B) for the stopper portion 100.

As shown in FIG. 7A, the notch 113 is disposed on the inside surface 111 on a side in the Y1 direction among the inside surfaces 111 that constitute the displacement permitting space 110 of the inner tube 70. The notch 113 opens downwardly and is contiguous with the displacement permitting space 110. When the withdrawal operation of the inner needle 12 is performed, the stopper portion 100 slides in a downward direction of the displacement permitting space 110, and by moving to the inner needle blocking position as shown in FIG. 7B, the claw member 105a becomes caught in the notch 113. Consequently, upward sliding of the stopper portion 100 is restricted, and the stopper portion 100 is placed in a waiting state at the inner needle blocking position. The structure of the lock mechanism 108 by which the stopper portion 100 is made to wait in the inner needle blocking position is not particularly limited. For example, the notch 113 may be formed on the stopper portion 100, and the hook part 105 may be formed on the inside surface 111.

The inner tube 70 having the above-described stopper portion 100, during manufacturing (for example, injection molding) thereof, as shown in FIG. 8, is formed in two members (first member 78, second member 79). Further, although in FIG. 8, for purposes of simplifying description, the first member 78 and the second member 79 are shown as being separated, actually, the proximal ends of both the first member 78 and the second member 79 are connected by a hinge mechanism 70a and injection-molded integrally. More specifically, distal end sides of the first member 78 and the second member 79 open vertically about the proximal end (hinge mechanism 70a). The first member 78 and the second member 79 may also be formed separately.

The first member 78 constitutes the upper side of the inner tube 70, and further includes the arms 74 in addition to the upper portion of the inner tube main body 72. The stopper portion 100 is formed on the side of the first member 78. In order to form the stopper portion 100, on the first member 78 around the periphery of the stopper portion 100, a space 78a which is wider than the displacement permitting space 110 is formed so that a non-illustrated forming mold can be inserted therein.

The second member 79 constitutes a lower part of the inner tube main body 72. In the proximal end tubular section 77 of the second member 79, at a position facing the aforementioned space 78a, two projecting walls 79a are formed as a pair. The two projecting walls 79a are of a thickness that enables them to be inserted into the space 78a of the first member 78, and mutually facing surfaces thereof form the inside surfaces 111 of the displacement permitting space 110. More specifically, the first member 78 and the second member 79 are of a spigot joint structure which, when assembled together mutually, render any unnecessary gaps therebetween as few as possible. In the case that the first member 78 and the second member 79 are assembled together, a cylindrical structure is constituted thereby, by the pair of projecting walls 79a of the second member 79 being inserted into gaps between the side walls that make up the space 78a of the first member 78 and the stopper portion 100 (see FIG. 7A). As a result, the stopper portion 100 is capable of sliding freely between the pair of projecting walls 79a with comparatively few gaps formed therebetween.

Further, the aforementioned sideways projection 112 is formed on the inside surface 111 of the projecting wall 79a in the Y2 direction, and the sideways projection 112 is inserted into the guide groove 106 of the stopper portion 100 (see FIG. 6A).

Furthermore, on the inside surface 111 on a proximal end side of the pair of projecting walls 79a, steps 114 are formed that slightly narrow the displacement permitting space 110 corresponding to the proximal end projecting part 104 of the stopper portion 100. The steps 114 are contiguous with the insertion hole 73 on lower end sides thereof. Therefore, in a state in which the stopper portion 100 waits in the inner needle blocking position, the proximal end side of the displacement permitting space 110 is substantially closed by the proximal end surface 101c of the stopper portion 100 and the proximal end projecting part 104, the steps 114 of the pair of projecting walls 79a, and an inner circumferential surface 73a of the insertion hole 73. The tip 12a of the inner needle 12 that is accommodated in the needle cover 40 faces toward the closed space, whereby advancement of the inner needle 12 is prevented.

The catheter assembly 10 according to the present embodiment can be constructed basically as described above. Next, descriptions of advantageous effects shall be given concerning assembly of the catheter assembly 10, and operations during use thereof.

During assembly of the catheter assembly 10, within the inner needle cover 40, the outer tube 80 and the adjoining tube 90 are first incorporated into the interior of the inner needle hub 20, and thereafter, the inner tube 70 is inserted from the distal end side outer tube 80. In addition, at a stage at which the proximal end tubular section 77 of the inner tube 70 has been inserted a certain degree into the outer tube side hollow portion 86 of the outer tube 80, the inner needle 12, with the catheter 14 and the catheter hub 30 arranged on a distal end side, is inserted therein from the distal end direction. At this stage, the distal end of the inner tube 70 is located more toward the distal end side than the distal end of the outer tube 80, the two arms 74 (in pairs) are expanded open, and the stopper portion 100 is arranged in the puncture standby position.

The inner needle 12 is inserted from the proximal end into the insertion hole 73 that opens on the distal end tubular section 76 of the inner tube 70, and having passed through the intermediate tubular section 75 and the proximal end tubular section 77, protrudes at the proximal end side of the inner tube 70. At this time, from the fact that the stopper portion 100 is in the puncture standby position, the inner needle 12 passes smoothly through the displacement permitting space 110. The proximal end of the inner needle 12 that protrudes on the proximal end side of the inner tube 70 is moved in the axial direction through the outer tube side hollow portion 86, and arrives at the retaining part 62 of the inner needle fixing member 60. The proximal end portion of the inner needle 12 is inserted into the interior of the inner needle fixing member 60 from the distal end of the retaining part 62, whereupon the inner needle 12 is fixedly retained in the retaining part 62 while penetrating through a non-illustrated packing provided inside the retaining part 62.

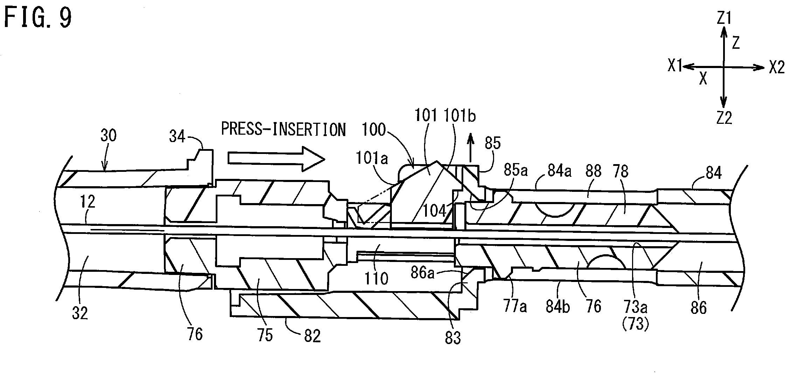

After fixing of the inner needle 12, the catheter hub 30 is attached to the distal end tubular section 76, and furthermore, by the inner tube 70 being press-inserted in the proximal end direction relative to the outer tube 80, the inner tube 70 is assembled into the outer tube 80. At this time, as shown in FIG. 9, the stopper portion 100 (and the inner tube side projection 77a) project more radially-outwardly than the opening 86a of the outer tube side hollow portion 86. However, due to the slits 87 (see FIG. 5B) being formed in the tubular portion 84 of the outer tube 80, at the time of insertion through the opening 86a, the stopper portion 100 causes the upper wall portion 84a and the distal end wall 85 of the tubular portion 84 to be displaced upwardly. Therefore, the stopper portion 100 (and the inner tube side projection 77a) is permitted elastically to be assembled therein in the proximal end direction.

Further, the distal end wall 85 of the outer tube 80 is equipped with the tapered surface 85a that is arranged to face toward the proximal end inclined surface 101b of the stopper portion 100. Therefore, when the stopper portion 100 moves in the proximal end direction, the proximal end inclined surface 101b and the tapered surface 85a come into contact, and displacement of the distal end wall 85 can be guided in an upward direction. When the stopper portion 100 passes beyond and exits from the distal end wall 85 in the proximal direction, the stopper portion 100 becomes disposed in the interior of the elongate hole 88.

Further, together with movement of the inner tube 70 in the proximal end direction, the arms 74 of the inner tube 70 become closed by the outer tube 80, whereby the protrusions 74d thereof become caught on the flange 34 of the catheter hub 30. Consequently, the catheter assembly 10 assumes a double tube structure of the inner needle 12 and the catheter 14, and the puncture enabled state is brought about in which the inner needle hub 20, the catheter hub 30, and the needle cover 40 are fitted together integrally. In the puncture enabled state, the catheter assembly 10 is subjected to an external environment protective means such as sterilization or the like, and then is supplied to a user.

When the user undertakes usage of the catheter assembly 10, the above-described puncture unit (the inner needle 12 and the catheter 14) are used integrally to puncture into the body of a patient. As shown in FIG. 5A, the stopper portion 100, which is in the puncture standby position, suppresses relative movement between the inner tube 70 and the outer tube 80, and by the inner needle 12 being pressed by the lower end surface of the stopper portion 100, vibrations or the like of the puncture unit can be suppressed.

After puncturing the patient, an operation to withdraw or retract the inner needle 12 is carried out. The user fixes the catheter hub 30 with one hand, and with the other hand grasps the inner needle hub 20 and moves the inner needle hub 20 in a direction to retract the inner needle 12. By the retracting movement of the inner needle hub 20, the hub main body 50 moves in the direction of retraction relatively with respect to the adjoining tube 90. In addition, when the hub main body 50 has been retracted a predetermined amount, the hub main body side protrusion 54 becomes caught on the adjoining tube outer side projection 96. Owing thereto, the adjoining tube 90 is pulled out by the hub main body 50, and undergoes a retracting movement relative to the outer tube 80. Further, when the adjoining tube 90 has been retracted a predetermined amount, the adjoining tube inner side projection 94 becomes caught on the outer tube side projection 89. Owing thereto, the outer tube 80 is pulled out by the adjoining tube 90, and undergoes a retracting movement relative to the inner tube 70.

As shown in FIGS. 5A and 5B, prior to the retracting movement of the hub main body 50, the adjoining tube 90, and the outer tube 80, the stopper portion 100 is in the puncture standby position, and vertical sliding of the stopper portion 100 is restricted by the inner needle 12. Therefore, the stopper portion 100 prevents separation and detachment (relative movement) of the inner tube 70 and the outer tube 80.

At a stage in which the adjoining tube 90 pulls out the outer tube 80, as shown in FIG. 3B, the tip 12a of the inner needle 12 assumes a state of having been moved toward the proximal end side more than the displacement permitting space 110. Therefore, the stopper portion 100 is capable of moving downwardly (in the X2 direction) of the inner tube 70 from the puncture standby position, and retracting movement of the outer tube 80 with respect to the inner tube 70 is permitted.

More specifically, when the outer tube 80 is moved and retracted relative to the inner tube 70, the hole edge (upper wall portion 84a) of the elongate hole 88 of the outer tube 80 abuts against the upper surface 102a (distal end inclined surface 101a) of the projecting piece 102 of the stopper portion 100. Therefore, accompanying the retracting movement of the outer tube 80, the upper wall portion 84a slides over the distal end inclined surface 101a of the stopper portion 100, and the stopper portion 100 is made to slide downwardly of the displacement permitting space 110. Thus, the stopper portion 100 is pressed downward to the inner needle blocking position, which enables passage through the interior of the outer tube side hollow portion 86 of the outer tube 80.

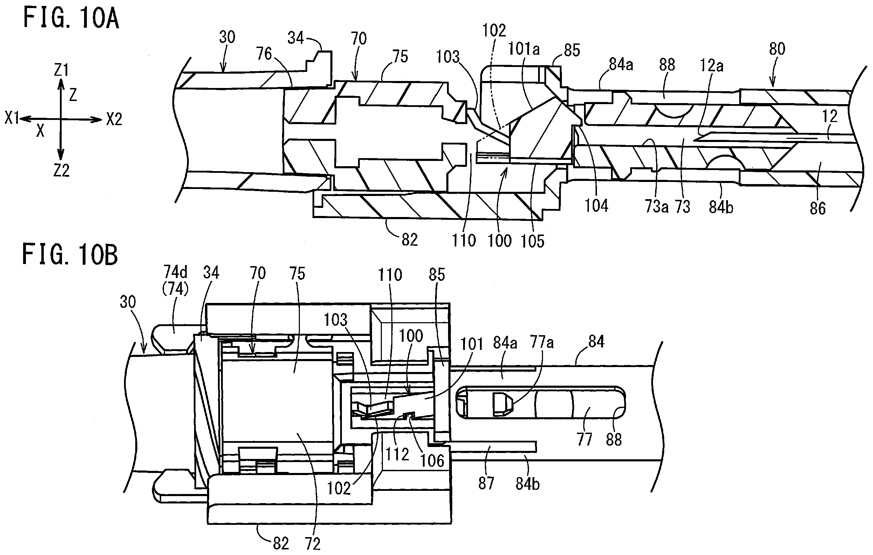

At a stage at which the outer tube 80 has been retracted a predetermined amount with respect to the inner tube 70, as shown in FIGS. 10A and 10B, the apex of the stopper portion 100 is inserted into the arm accommodating section 82 of the outer tube 80. At this stage, the hook part 105 of the stopper portion 100 becomes caught on the notch 113 of the inner tube 70, and the stopper portion 100 is maintained in the state of being disposed in the inner needle blocking position.

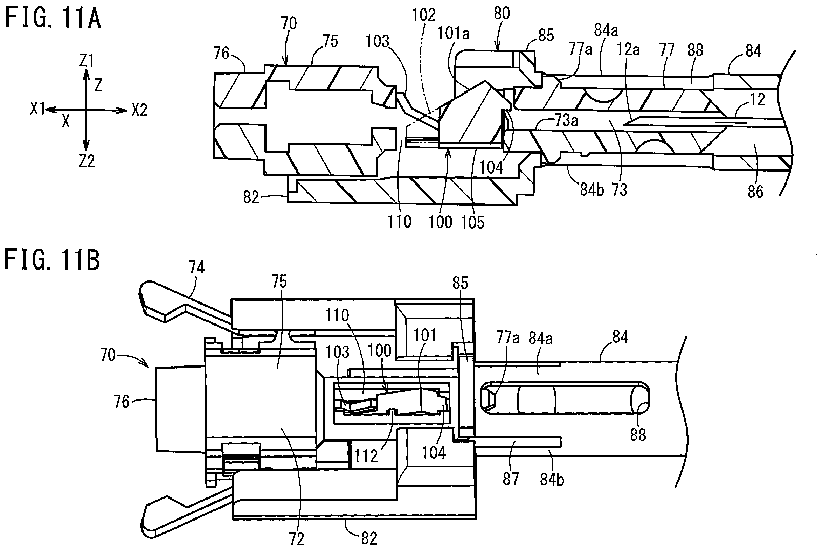

Upon further retracting movement of the outer tube 80, as shown in FIGS. 11A and 11B, the upper wall portion 84a of the outer tube 80 catches on the inner tube side projection 77a of the inner tube 70. Owing thereto, the inner tube 70 is pulled out by the outer tube 80 and moves in the proximal end direction. Further, at this stage, the two arms 74 (in pairs) are released from the arm accommodating section 82 and open outwardly in the widthwise direction. Thus, the catheter hub 30 that is caught on the protrusions 74d separates away smoothly from the needle cover 40, while the catheter 14 and the catheter hub 30 are placed in an indwelling state on the side of the patient. After placement thereof, an infusion tube is connected to the catheter hub 30.

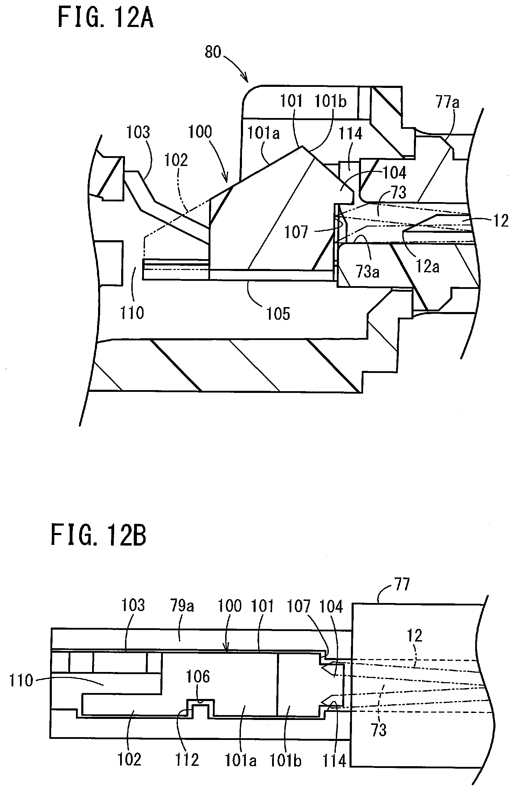

On the other hand, the inner needle 12, the inner needle hub 20, and the needle cover 40, which have been separated, are subject to a medical waste disposal process. At this time, the inner needle hub 20 and the needle cover 40 accommodate the inner needle 12 that was used for puncturing, such that the inner needle 12 cannot protrude to the exterior. More specifically, as shown in FIGS. 12A and 12B, by arranging the stopper portion 100 in the inner needle blocking position, the distal end side of the insertion hole 73 of the proximal end tubular section 77, which is an advancement path for the inner needle 12, is blocked. Therefore, even if the inner needle hub 20, etc., is pressed out in the distal end direction of the inner tube 70, advancement of the inner needle 12 can be blocked by the facing surface 107 of the stopper portion 100.

In particular, concerning the accommodating parts of the tip 12a, the facing surface 107 blocks the X1 direction of the inner needle 12, the proximal end projecting part 104 covers the Z1 direction, the steps 114 cover the Y direction on both sides, and the inner circumferential surface 73a of the insertion hole 73 covers the Z2 direction. Therefore, any gaps by which coming out of the inner needle 12 in the distal end direction could occur are eliminated, and advancement of the inner needle 12 is suitably blocked. Further, by the stopper portion 100 becoming caught on the guide groove 106 and the sideways protrusion 112, since movement in the axial direction (X direction) of the inner tube 70 is restricted, even if a pressing force (advancing force) is received from the inner needle 12, the blocked condition can be maintained.

As described above, in the catheter assembly 10 of the present embodiment, the stopper portion 100 that prevents relative movement between the inner tube 70 and the outer tube 80 slides and is arranged at the inner needle blocking position upon withdrawal of the inner needle 12. Therefore, advancement of the inner needle 12 can suitably be prevented by the stopper portion 100. Further, by the inner tube 70 of the catheter assembly 10 having the stopper portion 100 which is formed integrally therewith, there is no need for the member that blocks the inner needle 12 to be prepared (manufactured) as a separate member, and assembly thereof to the interior of the inner tube 70 is rendered unnecessary. Therefore, the production cost for the catheter assembly 10 can be reduced significantly, the assembly operation can be simplified, and production efficiency can be enhanced.

In this case, by the stopper portion 100 having the distal end inclined surface 101a, during relative movement of the outer tube 80, the stopper portion 100 slides smoothly along the distal end inclined surface 101a that is in contact with the upper wall portion 84a. Thus, the operation to withdraw the inner needle 12 can easily be carried out. Further, by the projecting piece 102 of the stopper portion 100 projecting out in the vicinity of the opening 86a (upper wall portion 84a) of the outer tube side hollow portion 86 of the outer tube 80, at the start of relative movement of the outer tube 80, the distal end inclined surface 101a can reliably be brought into contact with the upper wall portion 84a. Consequently, sliding of the stopper portion 100 can be guided more easily.

Further, by connecting the inner tube 70 and the stopper portion 100 by way of the connecting part 103, integral formation and assembly of the inner tube 70 can easily be performed. The connecting part 103 includes the hinge mechanisms 103a, whereby the stopper portion 100 is capable of sliding suitably with respect to the inner tube 70. Furthermore, in the catheter assembly 10, by the guide groove 106 being formed on the stopper portion 100, and by the sideways protrusion 112 being formed on the inside surface 111 of the displacement permitting space 110, an inclination or tilting in the posture of the stopper portion 100 during sliding thereof can be prevented. It goes without saying that the protrusion may be provided on the stopper portion 100, and the groove may be provided on the inside surface 111.

Still further, as a result of the lock mechanism 108, in the catheter assembly 10, after sliding thereof, the stopper portion 100 can reliably be made to continue waiting in the inner needle blocking position. Thus, flying out, etc., of the inner needle 12 that is accommodated in the needle cover 40 can reliably be blocked. Further still, by assembling the inner tube 70 that is constituted by the first member 78 and the second member 79, the projecting walls 79a are made to surround the periphery of the stopper portion 100, whereby gaps of the displacement permitting space 110 become reduced, and flying out of the inner needle 12 can more reliably be prevented.

Although a preferred embodiment of the present invention has been described, the present invention is not limited to the above-described embodiment. It goes without saying that various modifications can be adopted therein without departing from the scope of the invention.

## Claims

1. A catheter assembly (10) comprising:
an inner needle (12);
a catheter (14) through which the inner needle (12) extends;
a catheter hub (30) configured to retain the catheter (14); and
a needle cover (40) that accommodates the inner needle (12) when the inner needle (12) is withdrawn from the catheter (14), **characterized in that** the needle cover comprises:
an inner tube (70) configured to be attached to and detached from the catheter hub (30), the inner tube (70) comprising a stopper portion (100), and
an outer tube (80) configured to cover at least a portion of the inner tube (70), and to move relative to the inner tube (70) when the inner needle (12) is withdrawn,
wherein the stopper portion (100) is configured to prevent relative movement between the inner tube (70) and the outer tube (80) by being disposed in a standby position proximate to a radially outer side of the inner tube (70), in a state in which the inner needle (12) extends through the catheter (14), and
wherein the stopper portion (100) is configured to slide to an inner needle blocking position accompanying relative movement of the outer tube (80) with respect to the inner tube (70) when the inner needle (12) is withdrawn.

2. The catheter assembly (10) according to claim 1, wherein the stopper portion (100) includes an inclined surface (101a) that is inclined with respect to an axial direction of the inner tube (70), and the inclined surface (101a) is configured to slide to the inner needle blocking position by coming into contact with and being pressed by a portion of the outer tube (80) when there is accompanying relative movement of the outer tube (80) with respect to the inner tube (70).

3. The catheter assembly (10) according to claim 2, wherein:
the outer tube (80) includes a hollow portion (86) through which the inner tube (70) is inserted; and
the inclined surface (101a) is configured to be inserted into the hollow portion (86) in a state in which the stopper portion (100) is disposed in the standby position.

4. The catheter assembly (10) according to claim 3, wherein:
the stopper portion (100) includes a reverse side inclined surface (101b) disposed at an opposite position to the inclined surface (101a), so that in a state in which the inner needle (12) extends through the catheter (14), sliding of the stopper portion (100) radially-inwardly from the standby position is configured to be restricted by the inner needle (12); and
the outer tube (80) comprises a wall portion (85) configured to be displaceable radially-outwardly with respect to the outer tube (80) on a more distal end side than the stopper portion (100), and the wall portion (85) includes an outer tube side inclined surface (85a) that is substantially parallel with the reverse side inclined surface (101b).

5. The catheter assembly (10) according to claim 1, wherein the inner tube (70) and the stopper portion (100) are connected by a connecting part (103) including a hinge mechanism (103a) that is configured to allow the stopper portion (100) to freely slide.

6. The catheter assembly (10) according to claim 1, wherein:
a groove (106) that extends along a direction in which the stopper portion (100) slides is formed on one of the inner tube (70) and the stopper portion (100); and
a protrusion (112), which is inserted into the groove (106), is formed on another of the inner tube (70) and the stopper portion (100).

7. The catheter assembly (10) according to claim 1, wherein a lock mechanism (108) configured to maintain a displaced condition when the stopper portion (100) has slid to the inner needle blocking position, is disposed in the inner tube (70) and the stopper portion (100).

8. The catheter assembly (10) according to any one of claims 1 to 7, wherein:
the inner tube (70) comprises the stopper portion (100), a first member (78) that includes the stopper portion (100) and a space (78a) that surrounds the stopper portion (100) in a non-contacting manner, and a second member (79) that is attached to the first member (78); and
the second member (79) includes projecting walls (79a) that are accommodated in the space (78a) so that gaps on both sides of the stopper portion (100) are narrow in width.

9. The catheter assembly (10) according to claim 8, wherein:
the stopper portion (100) includes a projecting part (104) that surrounds a portion of an accommodating space (73) for the inner needle (12) in the inner needle blocking position; and
the projecting walls (79a) are arranged so that gaps on both sides of the projecting part (104) also are narrow in width.

## Patentansprüche

1. Katheteranordnung (10), umfassend:
eine innere Nadel (12),
einen Katheter (14), durch den sich die innere Nadel (12) erstreckt;
eine Katheternabe (30), die dazu ausgebildet ist, den Katheter (14) festzuhalten; und
eine Nadelabdeckung (40), die die innere Nadel (12) aufnimmt, wenn die innere Nadel (12) aus dem Katheter (14) herausgezogen wird,
**dadurch gekennzeichnet, dass** die Nadelabdeckung Folgendes umfasst:
ein inneres Rohr (70), das an der Katheternabe (30) befestigt und von dieser gelöst werden kann, wobei das innere Rohr (70) einen Anschlagabschnitt (100) umfasst, und
ein äußeres Rohr (80), das mindestens einen Abschnitt des inneren Rohres (70) bedecken kann und sich relativ zu dem inneren Rohr (70) bewegen kann, wenn die innere Nadel (12) herausgezogen wird,
wobei der Anschlagabschnitt (100) eine Relativbewegung zwischen dem inneren Rohr (70) und dem äußeren Rohr (80) verhindern kann, da er sich in einem Zustand, in dem sich die innere Nadel (12) durch den Katheter (14) erstreckt, in einer Standby-Position in der Nähe einer radial äußeren Seite des inneren Rohres (70) befindet, und
wobei der Anschlagabschnitt (100) in Verbindung mit einer Relativbewegung des äußeren Rohres (80) in Bezug zu dem inneren Rohr (70) zu einer die innere Nadel blockierenden Position gleiten kann, wenn die innere Nadel (12) herausgezogen wird.

2. Katheteranordnung (10) nach Anspruch 1, wobei der Anschlagabschnitt (100) eine geneigte Fläche (101a) aufweist, die in Bezug zu einer Axialrichtung des inneren Rohres (70) geneigt ist, und wobei die geneigte Fläche (101a) zu der die innere Nadel blockierenden Position gleiten kann, indem sie mit einem Abschnitt des äußeren Rohres (80) in Kontakt kommt und von diesem gedrückt wird, wenn damit eine Relativbewegung des äußeren Rohres (80) in Bezug zu dem inneren Rohr (70) verbunden ist.

3. Katheteranordnung (10) nach Anspruch 2, wobei:
das äußere Rohr (80) einen hohlen Abschnitt (86) aufweist, durch den das innere Rohr (70) eingesetzt wird; und
die geneigte Fläche (101a) in einem Zustand, in dem sich der Anschlagabschnitt (100) in der Standby-Position befindet, in den hohlen Abschnitt (86) eingesetzt werden kann.

4. Katheteranordnung (10) nach Anspruch 3, wobei:
der Anschlagabschnitt (100) auf der Rückseite eine geneigte Fläche (101b) aufweist, die an einer entgegengesetzten Position zu der geneigten Fläche (101a) angeordnet ist, so dass in einem Zustand, in dem sich die innere Nadel (12) durch den Katheter (14) erstreckt, die Gleitbewegung des Anschlagabschnitts (100) radial einwärts von der Standby-Position durch die innere Nadel (12) eingeschränkt werden kann; und
das äußere Rohr (80) einen Wandabschnitt (85) umfasst, der auf einer Stirnseite, die mehr distal gelegen ist als der Anschlagabschnitt (100), radial auswärts in Bezug zu dem äußeren Rohr (80) verschoben werden kann, und der Wandabschnitt (85) eine geneigte Fläche (85a) auf der Seite des äußeren Rohres aufweist, die im Wesentlichen parallel zu der auf der Rückseite befindlichen geneigten Fläche (101b) ist.

5. Katheteranordnung (10) nach Anspruch 1, wobei das innere Rohr (70) und der Anschlagabschnitt (100) durch ein Verbindungsteil (103) mit einem Scharniermechanismus (103a) verbunden sind, der dazu ausgebildet ist, den Anschlagabschnitt (100) frei gleiten zu lassen.

6. Katheteranordnung (10) nach Anspruch 1, wobei:
eine Nut (106), die sich entlang einer Richtung erstreckt, in der der Anschlagabschnitt (100) gleitet, auf einem von dem inneren Rohr (70) und dem Anschlagabschnitt (100) ausgebildet ist; und
ein Vorsprung (112), der in die Nut (106) eingesetzt wird, auf dem anderen von dem inneren Rohr (70) und dem Anschlagabschnitt (100) ausgebildet ist.

7. Katheteranordnung (10) nach Anspruch 1, wobei ein Verriegelungsmechanismus (108), der dazu ausgebildet ist, einen verschobenen Zustand zu halten, wenn der Anschlagabschnitt (100) zu der die innere Nadel blockierenden Position geglitten ist, in dem inneren Rohr (70) und dem Anschlagabschnitt (100) angeordnet ist.

8. Katheteranordnung (10) nach einem der Ansprüche 1 bis 7, wobei:
das innere Rohr (70) den Anschlagabschnitt (100), ein erstes Element (78), das den Anschlagabschnitt (100) aufweist, und einen Raum (78a), der den Anschlagabschnitt (100) berührungsfrei umgibt, sowie ein zweites Element (79) umfasst, das an dem ersten Element (78) befestigt ist; und
das zweite Element (79) vorspringende Wände (79a) aufweist, die in dem Raum (78a) aufgenommen werden, so dass Lücken auf beiden Seiten des Anschlagabschnitts (100) eine geringe Breite aufweisen.

9. Katheteranordnung (10) nach Anspruch 8, wobei:
der Anschlagabschnitt (100) einen vorspringenden Teil (104) aufweist, der in der die innere Nadel blockierenden Position einen Abschnitt eines Aufnahmeraums (73) für die innere Nadel (12) umschließt; und
die vorspringenden Wände (79a) so angeordnet sind, dass Lücken auf beiden Seiten des vorspringenden Teils (104) ebenfalls eine geringe Breite aufweisen.

## Revendications

1. Ensemble de cathéter (10) comprenant :
une aiguille intérieure (12) ;
un cathéter (14) à travers lequel s'étend l'aiguille intérieure (14) ;
un axe de cathéter (30) conçu pour retenir le cathéter (14) ; et
une enveloppe d'aiguille (40) qui reçoit l'aiguille intérieure (12) lorsque l'aiguille intérieure (12) est retirée du cathéter (14),
**caractérisé en ce que** l'enveloppe d'aiguille (40) comprend :
un tube intérieur (70) conçu pour être attaché et détaché de l'axe du cathéter (30), le tube intérieur (70) comprenant une partie de butée (100), et
un tube extérieur (80) conçu pour couvrir au moins une partie du tube intérieur (70), et pour se déplacer par rapport au tube intérieur (70) lorsque l'aiguille intérieure (12) est retirée,
où la partie de butée (100) est conçue pour empêcher un mouvement relatif entre le tube intérieur (70) et le tube extérieur (80) en étant disposée dans une position d'attente près d'un côté radialement extérieur du tube intérieur (70), dans un état où l'aiguille intérieure (12) s'étend à travers le cathéter (14), et
où la partie de butée (100) est conçue pour coulisser vers une position de blocage d'aiguille intérieure accompagnant le mouvement relatif du tube extérieur (80) par rapport au tube intérieur (70) lorsque l'aiguille intérieure (12) est retirée.

2. Ensemble de cathéter (10) selon la revendication 1, dans lequel la partie de butée (100) comprend une surface inclinée (101a) qui est inclinée par rapport à une direction axiale du tube intérieur (70), et la surface inclinée (101a) est conçue pour coulisser vers la position de blocage d'aiguille intérieure en venant en contact et en étant pressée par une partie du tube extérieur (80) lorsqu'il y a un mouvement relatif d'accompagnement du tube extérieur (80) par rapport au tube intérieur (70).

3. Ensemble de cathéter (10) selon la revendication 2, dans lequel :
le tube extérieur (80) comprend une partie creuse (86) à travers laquelle est inséré le tube intérieur (70) ; et
la surface inclinée (101a) est conçue pour être insérée dans la partie creuse (86) dans un état dans lequel la partie de butée (100) est placée dans la position d'attente.

4. Ensemble de cathéter (10) selon la revendication 3, dans lequel :
la partie de butée (100) comprend une surface inclinée côté inverse (101b) placée dans une position opposée à la surface inclinée (101a), de sorte que dans un état où l'aiguille intérieure (12) s'étend à travers le cathéter (14), le coulissement de la partie de butée (100) radialement vers l'intérieur depuis la position d'attente est prévu pour être limité par l'aiguille intérieure (12) ; et
le tube extérieur (80) comprend une partie de paroi (85) conçue pour pouvoir se déplacer radialement vers l'extérieur par rapport au tube extérieur (80) sur un côté d'extrémité plus distale que la partie de butée (100), et la partie de paroi (85) comprend une surface inclinée côté tube extérieur (85a) qui est sensiblement parallèle à la surface inclinée côté inverse (101b).

5. Ensemble de cathéter (10) selon la revendication 1, dans lequel le tube intérieur (70) et la partie de butée (100) sont reliés par une partie de liaison (103) comprenant un mécanisme d'articulation (103a) qui est conçu pour permettre à la partie de butée (100) de coulisser librement.

6. Ensemble de cathéter (10) selon la revendication 1, dans lequel :
une rainure (106) qui s'étend le long d'une direction dans laquelle coulisse la partie de butée (100) est formée sur l'un du tube intérieur (70) et de la partie de butée (100) ; et
une saillie (112), qui est insérée dans la rainure (106), est formée sur un autre du tube intérieur (70) et de la partie de butée (100).

7. Ensemble de cathéter (10) selon la revendication 1, dans lequel un mécanisme de blocage (108) conçu pour maintenir un état déplacé lorsque la partie de butée (100) a coulissé dans la position de blocage d'aiguille intérieure, est disposé dans le tube intérieur (70) et dans la partie de butée (100).

8. Ensemble de cathéter (10) selon l'une quelconque des revendications 1 à 7, dans lequel :
le tube intérieur (70) comprend la partie de butée (100), un premier élément (78) qui comprend la partie de butée (100) et un espace (78a) qui entoure la partie de butée (100) sans contact, et un second élément (79) qui est fixé au premier élément (78) ; et
le second élément (79) comprend des parois saillantes (79a) qui sont logées dans l'espace (78a) de sorte que des intervalles sur les deux côtés de la partie de butée (100) ont une largeur étroite.

9. Ensemble de cathéter (10) selon la revendication 8, dans lequel :
la partie de butée (100) comprend une partie saillante (104) qui entoure une partie d'un espace de réception (73) pour l'aiguille intérieure (12) dans la position de blocage d'aiguille intérieure ; et
les parois saillantes (79a) sont disposées de sorte que des espaces sur les deux côtés de la partie saillante (104) ont également une largeur étroite.
